(19) Europäisches Patentamt
European Patent Office
Office européen des brevets

(11) Publication number: **0 687 473 A1**

(12) **EUROPEAN PATENT APPLICATION
published in accordance with Art. 158(3) EPC**

(21) Application number: **94907082.5**

(22) Date of filing: **21.02.94**

(86) International application number:
**PCT/JP94/00261**

(87) International publication number:
**WO 94/20143 (15.09.94 94/21)**

(51) Int. Cl.6: **A61K 47/02**, A61K 47/20, A61K 47/22, A61K 47/34, A61K 31/55, //C07D487/06, C07D498/06

(30) Priority: **03.03.93 JP 42430/93**

(43) Date of publication of application:
**20.12.95 Bulletin 95/51**

(84) Designated Contracting States:
**AT BE CH DE DK ES FR GB GR IE IT LI LU NL PT SE**

(71) Applicant: **FUJISAWA PHARMACEUTICAL CO., LTD.**
**4-7, Doshomachi 3-chome**
**Chuo-ku**
**Osaka-shi**
**Osaka 541 (JP)**

(72) Inventor: **FUKUYAMA, Shinichi**
**13-27-710, Kusonoki-cho**
**Ashiya-shi,**
**Hyogo 659 (JP)**
Inventor: **MOROKOSHI, Noritsugu**
**6-22-26-322, Takadono,**
**Asahi-ku**
**Osaka-shi,**
**Osaka 535 (JP)**
Inventor: **NAKASHIMA, Keiichi**
**6-11-15, Higashitokiwadai,**
**Toyono-cho**
**Toyono-gun,**
**Osaka 563-01 (JP)**
Inventor: **KODA, Shigetaka**
**3-2-33, Higashishigigaoka,**
**Sango-cho**
**Ikoma-gun,**
**Nara 636 (JP)**

(74) Representative: **Türk, Dietmar, Dr. rer. nat.**
**Türk, Gille, Hrabal, Leifert**
**Patentanwälte**
**Brucknerstrasse 20**
**D-40593 Düsseldorf (DE)**

(54) **OPTICAL ISOMERIZATION INHIBITOR**

(57) An inhibitor comprising an antioxidant and usable for inhibiting the optical isomerization of optically active drugs.

EP 0 687 473 A1

## Technical Field

This invention relates to an optical isomerization inhibitor and to a pharmaceutical composition containing the same, and finds application in the medical field.

## Background Art

It is not known that an antioxidant is useful for inhibiting the optical isomerization of drugs having optical activity.

## Disclosure of the Invention

As far as any drug having optical activity is concerned, its optical isomer is regarded as impurity. The object of this invention is to inhibit the optical isomerization of drugs having optical activity.

After intensive research, the inventors of this invention found that antioxidants inhibit the optical isomerization of optically active drugs. This invention has been developed on the basis of the above discovery.

It is, therefore, an object of the invention to provide an optical isomerization inhibitor comprising an antioxidant.

Another object of this invention is to provide a pharmaceutical composition comprising an optically active drug and, as an inhibitor of its optical isomerization, an antioxidant.

The optically active drug for use in this invention is not critical in kind but includes, inter alia, optically active forms of compounds of the following general formula (I) and pharmaceutically acceptable salts thereof :

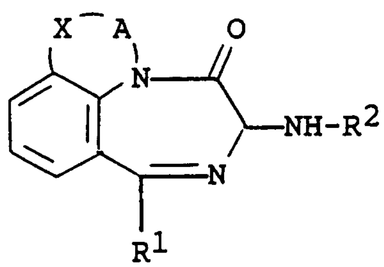

(I)

[wherein

$R^1$ is aryl which may have one or more suitable substituent(s);

X is -O- or

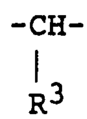

(wherein $R^3$ is hydrogen or lower alkyl);

A is a bond or lower alkylene which may have lower alkyl group(s);

$R^2$ is hydrogen or an acyl group].

It is known that the compound (I) has cholecystokinin-antagonizing activity and is of use as a prophylactic and/or therapeutic agent for pancreatitis and other diseases (Jpn. Kokai Tokkyo Koho JP2-111774).

Suitable pharmaceutically acceptable salts of an optically active form of the compound (I) are conventional non-toxic salts and include a metal salt such as an alkali metal salt (e.g. sodium salt, potassium salt, etc.) and an alkaline earth metal salt (e.g. calcium salt, magnesium salt, etc.), an ammonium salt, an organic base salt (e.g. trimethylamine salt, triethylamine salt, pyridine salt, picoline salt, dicyclohexylamine salt, N,N'-dibenzylethylenediamine salt, etc.), an organic acid salt (e.g. acetate, maleate, tartrate, methanesulfonate, benzenesulfonate, formate, toluenesulfonate, trifluoroacetate, etc.), an inorganic acid salt (e.g. hydrochloride, hydrobromide, sulfate, phosphate, etc.), a salt with an amino acid (e.g. arginine, aspartic acid, glutamic acid, etc.), and the like.

In the above and subsequent descriptions of the present specification, suitable examples and illustrations of the various definitions which the present invention include within the scope thereof are explained in detail as follows.

The term "lower" is intended to mean 1 to 6 carbon atom(s), unless otherwise indicated.

The term "higher" is intended to mean 7 to 20 carbon atoms, unless otherwise indicated.

Suitable "aryl" for $R^1$ may include phenyl, naphthyl and the like, and said aryl group may have one or more (preferably 1 to 3) suitable substituent(s) such as halogen, amino, lower alkoxy, mono(or di or tri)-halo-(lower)alkyl or the like.

Suitable "halogen" and "halogen moiety" in the term "mono(or di or tri)halo(lower)alkyl" may include chlorine, bromine, fluorine and iodine.

Suitable "lower alkoxy" may include methoxy, ethoxy, propoxy, isopropoxy, butoxy, isobutoxy, t-butoxy, pentyloxy, t-pentyloxy, hexyloxy and the like.

Suitable "lower alkylene" may include straight one having 1 to 6 carbon atom(s), such as methylene, ethylene, trimethylene, tetramethylene, pentamethylene or hexamethylene, preferably one having 1 to 3 carbon atom(s), and said lower alkylene group may have one or more (preferably 1 to 3) lower alkyl group-(s).

Suitable "lower alkyl" and "lower alkyl moiety" in the term "mono(or di or tri)halo(lower)alkyl" may include straight or branched one having 1 to 6 carbon atom(s), such as methyl, ethyl, propyl, isopropyl, butyl, isobutyl, sec-butyl, t-butyl, pentyl, t-pentyl, hexyl or the like, preferably one having 1 to 4 carbon atom(s).

Suitable "acyl" may include carbamoyl, aliphatic acyl group and acyl group containing an aromatic ring, which is referred to as aromatic acyl, or heterocyclic ring, which is referred to as heterocyclic acyl.

Suitable example of said acyl may be illustrated as follows :-

Carbamoyl;

Aliphatic acyl such as lower or higher alkanoyl (e.g. formyl, acetyl, propanoyl, butanoyl, 2-methylpropanoyl, pentanoyl, 2,2-dimethylpropanoyl, hexanoyl, heptanoyl, octanoyl, nonanoyl, decanoyl, undecanoyl, dodecanoyl, tridecanoyl, tetradecanoyl, pentadecanoyl, hexadecanoyl, heptadecanoyl, octadecanoyl, nonadecanoyl, icosanoyl, etc.);

lower or higher alkoxycarbonyl (e.g. methoxycarbonyl, ethoxycarbonyl, t-butoxycarbonyl, t-pentyloxycarbonyl, heptyloxycarbonyl, etc.);

lower or higher alkanesulfonyl (e.g. methanesulfonyl, ethanesulfonyl, etc.);

lower or higher alkoxysulfonyl (e.g. methoxysulfonyl, ethoxysulfonyl, etc.); or the like;

Aromatic acyl such as

aroyl (e.g. benzoyl, toluoyl, naphthoyl, etc.);

ar(lower)alkanoyl [e.g. phenyl(lower)alkanoyl (e.g. phenylacetyl, phenylpropanoyl, phenylbutanoyl, phenylisobutylyl, phenylpentanoyl, phenylhexanoyl, etc.), naphthyl(lower)alkanoyl (e.g. naphthylacetyl, naphthylpropanoyl, naphthylbutanoyl, etc.), etc.];

ar(lower)alkenoyl [e.g. phenyl(lower)alkenoyl (e.g. phenylpropenoyl, phenylbutenoyl, phenylmethacryloyl, phenylpentenoyl, phenylhexenoyl, etc.), naphthyl(lower)alkenoyl (e.g. naphthylpropenoyl, naphthylbutenoyl, naphthylpentenoyl, etc.), etc.];

ar(lower)alkoxycarbonyl [e.g. phenyl(lower)alkoxycarbonyl (e.g. benzyloxycarbonyl, etc.), etc.];

aryloxycarbonyl (e.g. phenoxycarbonyl, naphthyloxycarbonyl, etc.);

aryloxy(lower)alkanoyl (e.g. phenoxyacetyl, phenoxypropionyl, etc.);

arylcarbamoyl (e.g. phenylcarbamoyl, etc.);

arylthiocarbamoyl (e.g. phenylthiocarbamoyl, etc.);

arylglyoxyloyl (e.g. phenylglyoxyloyl, naphthylglyoxyloyl, etc.);

arenesulfonyl (e.g. benzenesulfonyl, p-toluenesulfonyl, etc.); or the like;

Heterocyclic acyl such as heterocycliccarbonyl;

heterocyclic(lower)alkanoyl (e.g. thienylacetyl, thienylpropanoyl, thienylbutanoyl, thienylpentanoyl, thienylhexanoyl, thiazolylacetyl, thiadiazolylacetyl, tetrazolylacetyl, etc.);

heterocyclic(lower)alkenoyl (e.g. heterocyclicpropenoyl, heterocyclicbutenoyl, heterocyclicpentenoyl, heterocyclichexenoyl, etc.;

heterocyclicglyoxyloyl (e.g. thiazolylglyoxyloyl, thienylglyoxyloyl, etc.); or the like;

in which suitable heterocyclic moiety in the terms "heterocycliccarbonyl", "heterocyclic(lower)alkanoyl", "heterocyclic(lower)alkenoyl" and "heterocyclicglyoxyloyl" as mentioned above means, in more detail, saturated or unsaturated, monocyclic or polycyclic heterocyclic group containing at least one hetero-atom such as an oxygen, sulfur, nitrogen atom and the like.

And, especially preferable heterocyclic group may be heterocyclic group such as

unsaturated 3 to 8-membered (more preferably 5 or 6-membered) heteromonocyclic group containing 1 to 4-nitrogen atom(s), for example, pyrrolyl, pyrrolinyl, imidazolyl, pyrazolyl, pyridyl and its N-oxide, dihydropyridyl, pyrimidyl, pyrazinyl, pyridazinyl, triazolyl (e.g. 4H-1,2,4-triazolyl, 1H-1,2,3-triazolyl, 2H-1,2,3-triazolyl, etc.), tetrazolyl (e.g. 1H-tetrazolyl, 2H-tetrazolyl, etc.), etc.;
saturated 3 to 8-membered (more preferably 5 or 6-membered) heteromonocyclic group containing 1 to 4 nitrogen atom(s), for example pyrrolidinyl, imidazolidinyl, piperidino, piperazinyl, etc.;
unsaturated condensed heterocyclic group containing 1 to 4 nitrogen atom(s), for example, indolyl, isoindolyl, indolinyl, indolizinyl, benzimidazolyl, quinolyl, isoquinolyl, indazolyl, benzotriazolyl, etc.;
unsaturated 3 to 8-membered (more preferably 5 or 6-membered) heteromonocyclic group containing 1 to 2 oxygen atom(s) and 1 to 3 nitrogen atom(s), for example, oxazolyl, isoxazolyl, oxadiazolyl (e.g. 1,2,4-oxadiazolyl, 1,3,4-oxadiazolyl, 1,2,5-oxadiazolyl, etc.) etc.;
saturated 3 to 8-membered (more preferably 5 or 6-membered) heteromonocyclic group containing 1 to 2 oxygen atom(s) and 1 to 3 nitrogen atom(s), for example, morpholinyl, sydnonyl, etc.;
unsaturated condensed heterocyclic group containing 1 to 2 oxygen atom(s) and 1 to 3 nitrogen atom(s), for example, benzoxazolyl, benzoxadiazolyl, etc.;
unsaturated 3 to 8-membered (more preferably 5 or 6-membered) heteromonocyclic group containing 1 to 2 sulfur atom(s) and 1 to 3 nitrogen atom(s), for example, thiazolyl, isothiazolyl, thiadiazolyl (e.g. 1,2,3-thiadiazolyl, 1,2,4-thiadiazolyl, 1,3,4-thiadiazolyl, 1,2,5-thiadiazolyl, etc.), dihydrothiazinyl, etc.;
saturated 3 to 8-membered (more preferably 5 or 6-membered) heteromonocyclic group containing 1 to 2 sulfur atom(s) and 1 to 3 nitrogen atom(s), for example, thiazolidinyl, etc.;
unsaturated 3 to 8-membered (more preferably 5 or 6-membered) heteromonocyclic group containing 1 to 2 sulfur atom(s), for example, thienyl, dihydrodithiinyl, dihydrodithionyl, etc.;
unsaturated condensed heterocyclic group containing 1 to 2 sulfur atom(s) and 1 to 3 nitrogen atom(s), for example, benzothiazolyl, benzothiadiazolyl, etc.;
unsaturated 3 to 8-membered (more preferably 5 to 6-membered) heteromonocyclic group containing an oxygen atom, for example, furyl, etc.;
unsaturated 3 to 8-membered (more preferably 5 or 6-membered) heteromonocyclic group containing an oxygen atom and 1 to 2 sulfur atom(s), for example, dihydrooxathiinyl, etc.;
unsaturated condensed heterocyclic group containing 1 to 2 sulfur atom(s), for example, benzothienyl, benzodithiinyl, etc.;
unsaturated condensed heterocyclic group containing an oxygen atom and 1 to 2 sulfur atom(s), for example, benzoxathiinyl, etc. and the like.
The acyl moiety as stated above may have one to ten, same or different, suitable substituent(s) such as halogen (e.g. fluorine, chlorine, bromine or iodine), hydroxy, nitro, lower alkyl (e.g. methyl, ethyl, propyl, isopropyl, butyl, isobutyl, t-butyl, pentyl, hexyl, etc.);
amino, protected amino, heterocyclic(lower)alkylamino wherein heterocyclic and lower alkyl moieties can be referred to the ones as mentioned above, lower alkoxy (e.g. methoxy, ethoxy, propoxy, butoxy, t-butoxy, pentyloxy, hexyloxy, etc.), carboxy, protected carboxy,
N,N-di(lower)alkylamino(lower)alkyl (e.g. N,N-dimethylaminomethyl, N,N-diethylaminomethyl, N,N-dipropylaminomethyl, N,N-dimethylaminoethyl, N,N-diethylaminoethyl, N,N-dipropylaminoethyl, N,N-dimethylaminopropyl, N,N-diethylaminopropyl, N,N-dipropylaminopropyl, N,N-dibutylaminomethyl, N,N-dipentylaminomethyl, N,N-dihexylaminomethyl, etc.), hydroxyimino(lower)alkyl (e.g. hydroxyiminomethyl, hydroxyiminoethyl, hydroxyiminopropyl, hydroxyiminobutyl, hydroxyiminopentyl, hydroxyiminohexyl, etc.), arylimino(lower)alkyl [e.g. phenylimino(lower)alkyl (e.g. phenyliminomethyl, phenyliminoethyl, phenyliminopropyl, phenyliminobutyl, phenyliminopentyl, phenyliminohexyl, etc.), etc.], acyl such as lower alkanoyl (e.g. formyl, acetyl, propanoyl, butanoyl, pentanoyl, hexanoyl, etc.), hydroxy(lower)-alkylheterocyclic(lower)alkyl wherein lower alkyl and heterocyclic moieties can be referred to the ones as mentioned above, mono(or di or tri)-halo(lower)alkyl, arylamino (e.g. phenylamino, etc.), or the like.

Suitable "protected amino" may include acylamino and the like.

Suitable "acyl moiety" in the term "acylamino" can be referred to the ones as mentioned above.

Suitable "protected carboxy" may include esterified carboxy and the like.

Suitable example of the ester moiety of an esterified carboxy may be the ones such as lower alkyl ester (e.g. methyl ester, ethyl ester, propyl ester, isopropyl ester, butyl ester, isobutyl ester, tert-butyl ester, pentyl ester, hexyl ester, 1-cyclopropylethyl ester, etc.) which may have at least one suitable substituent(s), for example, lower alkanoyloxy(lower)alkyl ester [e.g. acetoxymethyl ester, propionyloxymethyl ester, butyryloxymethyl ester, valeryloxymethyl ester, pivaloyloxymethyl ester, hexanoyloxymethyl ester, 1(or 2)-acetoxyethyl ester, 1(or 2 or 3)-acetoxypropyl ester, 1(or 2 or 3 or 4)-acetoxybutyl ester, 1(or 2)-propionyloxyethyl ester, 1(or 2 or 3)-propionyloxypropyl ester, 1(or 2)-butyryloxyethyl ester, 1(or 2)-isobutyryloxyethyl ester, 1(or 2)-pivaloyloxyethyl ester, 1(or 2)-hexanoyloxyethyl ester, isobutyryloxymethyl ester, 2-ethylbutyryloxymethyl ester, 3,3-dimethylbutyryloxymethyl ester, 1(or 2)-pentanoyloxyethyl ester, etc.], lower alkanesulfonyl(lower)alkyl ester (e.g. 2-mesylethyl ester, etc.), mono(or di or tri)-halo(lower)alkyl ester (e.g. 2-iodoethyl ester, 2,2,2-trichloroethyl ester, etc.), lower alkoxycarbonyloxy(lower)alkyl ester (e.g. methoxycarbonyloxymethyl ester, ethoxycarbonyloxymethyl ester, 2-methoxycarbonyloxyethyl ester, 1-ethoxycarbonyloxyethyl ester, 1-isopropoxycarbonyloxyethyl ester, etc.), phthalidylidene(lower)alkyl ester, or (5-lower alkyl-2-oxo-1,3-dioxol-4-yl)(lower)alkyl ester [e.g. (5-methyl-2-oxo-1,3-dioxol-4-yl)methyl ester, (5-ethyl-2-oxo-1,3-dioxol-4-yl)methyl ester, (5-propyl-2-oxo-1,3-dioxol-4-yl)ethyl ester, etc.];
lower alkenyl ester (e.g. vinyl ester, allyl ester, etc.); lower alkynyl ester (e.g. ethynyl ester, propynyl ester, etc.);
ar(lower)alkyl ester which may have at least one suitable substituent(s) such as mono(or di or tri)phenyl-(lower)alkyl ester which may have at least one suitable substituent(s) (e.g. benzyl ester, 4-methoxybenzyl ester, 4-nitrobenzyl ester, phenethyl ester, trityl ester, benzhydryl ester, bis(methoxyphenyl)methyl ester, 3,4-dimethoxybenzyl ester, 4-hydroxy-3,5-di-tert-butylbenzyl ester, etc.);
aryl ester which may have at least one suitable substituent(s) (e.g. phenyl ester, 4-chlorophenyl ester, tolyl ester, tert-butylphenyl ester, xylyl ester, mesityl ester, cumenyl ester, etc.);
phthalidyl ester; and the like.

Preferred embodiments of an optically active form of the above compound (I) are as follows.

$R^1$ is phenyl which may have halogen,

X is -O- or

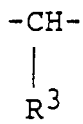

(in which $R^3$ is hydrogen or lower alkyl),

A is a bond or lower alkylene which may have lower alkyl,

$R^2$ is hydrogen;

ar(lower)alkenoyl which may have 1 to 3 substituent(s) selected from the group consisting of halogen, hydroxy, nitro, lower alkyl, lower alkoxy, amino, protected amino and heterocyclic(lower)alkylamino [more preferably phenyl(lower)alkenoyl which may have one or two substituent(s) selected from the group consisting of halogen, hydroxy, nitro, lower alkyl, lower alkoxy, amino, acylamino and imidazolyl(lower)-alkylamino, most preferably phenyl(lower)alkenoyl which may have one or two substituent(s) selected from the group consisting of halogen, hydroxy, nitro, lower alkyl, lower alkoxy, amino, lower alkanoylamino and imidazolyl(lower)alkylamino];

heterocyclic(lower)alkenoyl which may have carboxy or protected carboxy [more preferably indolyl-(lower)alkenoyl which may have carboxy or protected carboxy; imidazolyl(lower)alkenoyl; or quinolyl(lower)-alkenoyl, most preferably indolyl(lower)alkenoyl which may have carboxy or esterified carboxy; imidazolyl-(lower)alkenoyl; or quinolyl(lower)alkenoyl];

heterocycliccarbonyl which may have N,N-di(lower)-alkylamino(lower)alkyl, hydroxyimino(lower)alkyl, arylimino(lower)alkyl, acyl or hydroxy(lower)alkylheterocyclic(lower)alkyl [more preferably indolylcarbonyl which may have N,N-di(lower)alkylamino(lower)alkyl, hydroxyimino(lower)alkyl, phenylimino(lower)alkyl, lower alkanoyl or hydroxy(lower)alkylpiperazinyl(lower)alkyl;
quinolylcarbonyl; or indolinylcarbonyl];
aroyl which may have 1 to 3 substituent(s) selected from the group consisting of halogen, amino and mono-(or di or tri)halo(lower)alkyl [more preferably benzoyl which may have one or two substituent(s) selected from the group consisting of halogen, amino and mono(or di or tri)halo(lower)alkyl];

arylcarbamoyl which may have halogen or lower alkoxy [more preferably phenylcarbamoyl which may have halogen or lower alkoxy];

arylamino(lower)alkanoyl [more preferably phenylamino(lower)alkanoyl]; or

ar(lower)alkanoyl which may have amino or protected amino [more preferably phenyl(lower)alkanoyl which may have amino or acylamino, most preferably phenyl(lower)alkanoyl which may have amino, lower alkoxycarbonylamino or phenyl-thiocarbamoylamino].

Particularly preferred embodiments of the optically active drug for use in this invention are optically active forms of compounds of the following general formula (Ia) and pharmaceutically acceptable salts thereof :

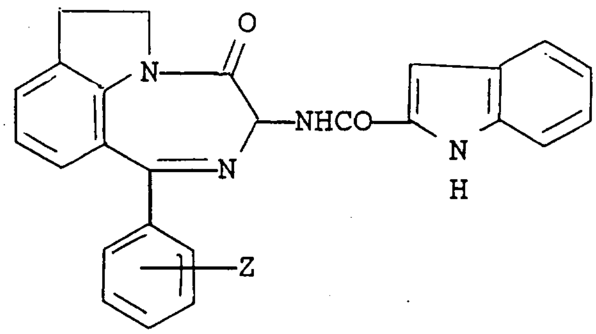

(Ia)

(wherein Z is hydrogen or halogen).

The preferable antioxidant for use as an optical isomerization inhibitor may include alkali metal sulfites (e.g. sodium sulfite, etc.), alkali metal bisulfites (e.g. sodium bisulfite, etc.), alkali metal pyrosulfites (e.g. sodium pyrosulfite, etc.), alkali metal thiosulfates (e.g. sodium thiosulfate, etc.), rongalite, ascorbic acid, isoascorbic acid, thioglycerol, thiosorbitol, cysteine hydrochloride, tocopherol (e.g. α-tocopherol, etc.), dibutyl hydroxy toluene (BHT), butyl hydroxyanisol (BHA), propyl gallate, nordihydroguaiaretic acid, ethanol, alanine (e.g. α-alanine, etc.), glycine, ubiquinones, carotenoids and the like.

There is no limitation on the dosage form in which the pharmaceutical composition of this invention can be provided. Thus, such dosage forms as tablet, powder, granule, hard capsule, soft capsule, syrup, solution, emulsion, suspension, elixir, injection, etc. can be mentioned. Particularly, the pharmaceutical composition in which the drug is in liquid is preferable.

The pharmaceutical composition of this invention may further contain organic and/or inorganic additives which are commonly used in pharmaceutical manufacture, e.g. excipients such as sucrose, starch, mannitol, sorbitol, lactose, glucose, cellulose, talc, calcium phosphate, calcium carbonate, etc., binders such as cellulose, methylcellulose, hydroxypropylcellulose, polypropylpyrrolidone, gelatin, gum arabic, polyethylene glycol, sucrose, starch, etc., disintegrators such as starch, carboxymethylcellulose, calcium carboxymethylcellulose, hydroxypropyl starch, glycol starch sodium, sodium hydrogen carbonate, calcium phosphate, calcium citrate, etc., lubricants such as magnesium stearate, talc, sodium lauryl sulfate, etc., flavoring agents such as citric acid, menthol, glycine, orange powder, etc., suspending agents such as methylcellulose, polyvinylpyrrolidone, aluminum stearate, etc., dispersing agents, solvents such as water, polyethylene glycol, etc.

The pharmaceutical composition according to this invention can be manufactured by formulating the optically active drug with the above-mentioned optical isomerization inhibitor (antioxidant) and other optional additives and processing the composition into an intended dosage form in the per se known manner.

The ratio of the optically active drug to the antioxidant (optical isomerization inhibitor) in the pharmaceutical composition of this invention need not be critical but can be selected liberally according to the species of drug and antioxidant used. Generally speaking, the weight ratio of the drug to the antioxidant is about 100:1 through about 1:1000, preferably about 50:1 through about 1:500 and, more preferably about 30:1 through about 1:200.

The following experiment demonstrates that anti-oxidants inhibit the optical isomerization of optically active drugs.

Method

The drug (FK480) was dissolved in PEG400 or PEG400-glycerin with stirring at 50 °C for a few hours. Then, various antioxidants, in appropriate amounts, were respectively added to the above solution and dissolved under sonication (suspended in the case of glycine and DL-α-alanine) to prepare test samples.

Each sample was taken in a test tube with ground stopper and allowed to stand under gas-tight conditions in a mini-jet oven at 60 °C for 7 or 14 days.

At the end of each storage period, the amount of optical isomer was determined by liquid chromatography. The computation formula for the amount of optical isomer is shown below.

$$\text{The amount of optical isomer (\%)} = \frac{\text{Peak area of optical isomer}}{\text{Peak area of optical isomer + peak area of FK480}} \times 100$$

Results

Table 1

| | Sample | Amount of optical isomer (%) | |
|---|---|---|---|
| | | Initial | 60 °C, 7 days |
| Control: | FK480 (2 mg)<br>PEG400 (313.2 mg)<br>Glycerin(34.8 mg) | 0.09 | 1.40 |
| (1) | FK480 (2 mg)<br>PEG400 (453.7 mg)<br>Ethanol(244.3 mg) | 0.07 | 0.28 |
| (2) | FK480 (2 mg)<br>PEG400 (349 mg)<br>Ethanol (349 mg) | 0.07 | 0.25 |
| (3) | FK480 (2 mg)<br>PEG400 (313.2 mg)<br>Glycerin(34.8 mg)<br>Glycine (3.4 mg) | 0.07 | 0.49 |

Table 2

| Sample | | Amount of optical isomer (%) | |
|---|---|---|---|
| | | Initial | 60°C, 14 days |
| Control: | FK480 (2 mg)<br>PEG400 (313.2 mg)<br>Glycerin(34.8 mg) | 0.09 | 3.84 |
| (4) | FK480 (2 mg)<br>PEG400 (313.2 mg)<br>Glycerin(34.8 mg)<br>DL-α-Alanine(0.4 mg) | 0.08 | 0.54 |
| (5) | FK480 (2 mg)<br>PEG400 (313.2 mg)<br>Glycerin(34.8 mg)<br>DL-α-Alanine(0.04 mg) | 0.08 | 0.43 |
| (6) | FK480 (2 mg)<br>PEG400 (349 mg)<br>Ethanol (349 mg) | 0.07 | 0.53 |

Table 2 (continued)

| | Sample | Amount of optical isomer (%) | |
|---|---|---|---|
| | | Initial | 60°C, 14 days |
| (7) | FK480 (2 mg)<br>PEG400 (313.2 mg)<br>Glycerin(34.8 mg)<br>DL-α-Tocopherol (2.0 mg) | 0.09 | 0.74 |
| (8) | FK480 (2 mg)<br>PEG400 (313.2 mg)<br>Glycerin(34.8 mg)<br>DL-α-Tocopherol (0.4 mg) | 0.09 | 0.68 |
| (9) | FK480 (2 mg)<br>PEG400 (313.2 mg)<br>Glycerin(34.8 mg)<br>BHT (1.0 mg) | 0.09 | 0.70 |
| (10) | FK480 (2 mg)<br>PEG400 (313.2 mg)<br>Glycerin(34.8 mg)<br>BHT (0.15 mg) | 0.09 | 0.69 |
| (11) | FK480 (2 mg)<br>PEG400 (313.2 mg)<br>Glycerin(34.8 mg)<br>Ascorbic acid (0.08 mg) | 0.09 | 0.95 |
| (12) | FK480 (2 mg)<br>PEG400 (313.2 mg)<br>Glycerin(34.8 mg)<br>Sodium pyrosulfite (0.9 mg) | 0.09 | 0.80 |
| (13) | FK480 (2 mg)<br>PEG400 (313.2 mg)<br>Glycerin(34.8 mg)<br>Sodium pyrosulfite (0.2 mg) | 0.09 | 0.64 |

*

- FK480 : (3S)-1-(2-Fluorophenyl)-3,4,6,7-tetrahydro-3-(2-indolylcarbonylamino)-4-oxopyrrolo[3,2,1-jk]-[1,4]benzodiazepine
- PEG400 : Polyethylene glycol 400
- BHT : Dibutyl hydroxy toluene

The above test results indicate that the antioxidants inhibit the optical isomerization of optically active drugs.

The following examples are intended to describe this invention in further detail and should by no means be considered to define the scope of the invention.

Example 1

After dl-α-tocopherol, polyethylene glycol 400 and concentrated glycerin are admixed, FK480 is added and dissolved with stirring to prepare a solution for contents of soft capsules. On the other hand, gelatin, concentrated glycerin, D-sorbitol solution, ethyl p-hydroxybenzoate and propyl p-hydroxybenzoate are admixed and dissolved with stirring to prepare a solution for shells of soft capsules.

The two solutions prepared as above were subjected to the conventional filling procedure to provide soft capsules.

Concentrated glycerin was added for stabilizing the shell.

The composition per capsule is as follows.

Soft capsule (1) (Size : No. 6 oval)

Contents

| | |
|---|---|
| FK480 | 0.5 mg |
| dl-α-Tocopherol | 0.2 mg |
| Polyethylene glycol 400 | 314.37 mg |
| Concentrated glycerin | 34.93 mg |
| | 350.00 mg |

Shell

| | |
|---|---|
| Gelatin | 133.31 mg |
| Concentrated glycerin | 46.80 mg |
| D-Sorbitol solution | 17.02 mg |
| Ethyl p-hydroxybenzoate | 0.58 mg |
| Propyl p-hydroxybenzoate | 0.29 mg |
| | 198.00 mg |

Soft capsule (2) (Size : No. 6 oval)

Contents

| | |
|---|---|
| FK480 | 1.0 mg |
| dl-α-Tocopherol | 5.0 mg |
| Polyethylene glycol 400 | 309.6 mg |
| Concentrated glycerin | 34.4 mg |
| | 350.00 mg |

Shell

Same as soft capsule (1)

Soft capsule (3) (Size : No. 4 oval)

Contents

| | |
|---|---|
| FK480<br>dl-α-Tocopherol<br>Polyethylene glycol 400<br>Concentrated glycerin | 0.5 mg<br>0.2 mg<br>209.07 mg<br>23.23 mg |
| | 233.00 mg |

Shell

| | |
|---|---|
| Gelatin<br>Concentrated glycerin<br>D-Sorbitol solution<br>Ethyl p-hydroxybenzoate<br>Propyl p-hydroxybenzoate | 88.87 mg<br>31.20 mg<br>11.35 mg<br>0.39 mg<br>0.19 mg |
| | 132.00 mg |

Soft capsule (4) (Size : No. 4 oval)

Contents

| | |
|---|---|
| FK480<br>dl-α-Tocopherol<br>Polyethylene glycol 400<br>Concentrated glycerin | 0.25 mg<br>0.20 mg<br>209.29 mg<br>23.26 mg |
| | 233.00 mg |

Shell

Same as soft capsule (3).

Example 2

Soft capsules are manufactured by the same procedure as Example 1 except that the samples (3) - (5) and (7) - (13) used in the test described hereinbefore are used in lieu of the contents of soft capsules (1) and (2) which were used in Example 1.

Example 3

FK480, polyethylene glycol 400 and dl-α-tocopherol are dissolved in the conventional manner to provide a solution for oral administration.

The composition of the solution is as follows.

| | |
|---|---|
| FK480<br>dl-α-Tocopherol<br>Polyethylene glycol 400 | 10 mg<br>10 mg<br>q.s. |
| | 50 ml |

**Claims**

1. An optical isomerization inhibitor for optically active drugs which comprises an antioxidant.

2. A pharmaceutical composition comprising the optical isomerization inhibitor of claim 1 and an optically active drug.

3. The pharmaceutical composition of claim 2 wherein the optically active drug is an optically active form of a compound of the following general formula or a pharmaceutically acceptable salt thereof.

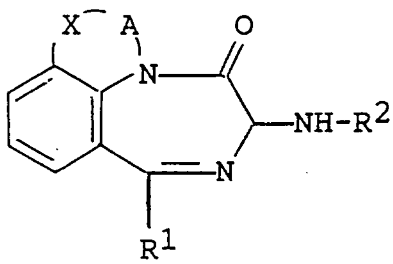

[wherein
$R^1$ is aryl which may have one or more suitable substituent(s);
X is -O- or

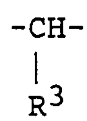

(wherein $R^3$ is hydrogen or lower alkyl);
A is a bond or lower alkylene which may have lower alkyl group(s);
$R^2$ is hydrogen or an acyl group].

4. The pharmaceutical composition of claim 3 wherein
$R^1$ is phenyl which may have halogen,
X is -O- or

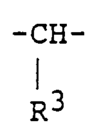

(wherein $R^3$ is hydrogen or lower alkyl),
A is a bond or lower alkylene which may have lower alkyl group(s); and
$R^2$ is hydrogen; phenyl(lower)alkenoyl which may have 1 or 2 substituent(s) selected from the group consisting of halogen, hydroxy, nitro, lower alkyl, lower alkoxy, amino, acylamino and imidazolyl(lower)alkylamino;
indolyl(lower)alkenoyl which may have carboxy or protected carboxy;

imidazolyl(lower)alkenoyl;
quinolyl(lower)alkenoyl; indolylcarbonyl which may have N,N-di(lower)alkylamino(lower)alkyl, hydroxyimino(lower)alkyl, phenylimino(lower)alkyl, lower alkanoyl or hydroxy(lower)-alkylpiperazinyl(lower)alkyl;
quinolylcarbonyl; indolinylcarbonyl; benzoyl which may have 1 or 2 substituent(s) selected from the group consisting of halogen, amino and mono(or di or tri)halo(lower)alkyl;
phenylcarbamoyl which may have halogen or lower alkoxy; phenylamino(lower)alkanoyl; or phenyl(lower)alkanoyl which may have amino or acylamino.

**5.** The pharmaceutical composition of claim 4 wherein
$R^2$ is hydrogen; phenyl(lower)alkenoyl which may have 1 or 2 substituent(s) selected from the group consisting of halogen, hydroxy, nitro, lower alkyl, lower alkoxy, amino, lower alkanoylamino and imidazolyl(lower)alkylamino;
indolyl(lower)alkenoyl which may have carboxy or esterified carboxy;
imidazolyl(lower)alkenoyl;
quinolyl(lower)alkenoyl; indolylcarbonyl which may have N,N-di(lower)alkylamino(lower)alkyl, hydroxyimino(lower)alkyl, phenylimino(lower)alkyl, lower alkanoyl or hydroxy(lower)-alkylpiperazinyl(lower)alkyl; quinolylcarbonyl; indolinylcarbonyl; benzoyl which may have 1 or 2 substituent(s) selected from the group consisting of halogen, amino and mono(or di or tri)-halo(lower)alkyl;
phenylcarbamoyl which may have halogen or lower alkoxy; phenylamino(lower)alkanoyl; or phenyl(lower)alkanoyl which may have amino, lower alkoxycarbonylamino or phenylthiocarbamoylamino.

**6.** The pharmaceutical composition of claim 3 wherein the optically active drug is an optically active form of a compound of the following general formula or a pharmaceutically acceptable salt thereof :

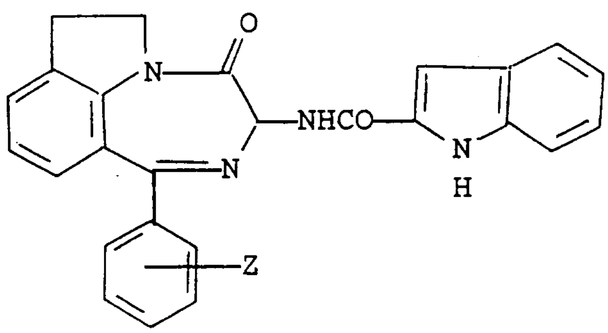

(wherein Z is hydrogen or halogen).

**7.** The pharmaceutical composition of claim 6 wherein the optically active drug is (3S)-1-(2-fluorophenyl)-3,4,6,7-tetrahydro-3-(2-indolylcarbonylamino)-4-oxopyrrolo[3,2,1-jk][1,4]benzodiazepine or a pharmaceutically acceptable salt thereof.

**8.** The pharmaceutical composition according to any of claims 2 through 7 wherein the weight ratio of the optically active drug to the optical isomerization inhibitor is 100:1 through 1:1000.

**9.** The pharmaceutical composition of claim 8 wherein the weight ratio of the optically active drug to the optical isomerization inhibitor is 50:1 through 1:500.

**10.** The pharmaceutical composition of claim 9 wherein the weight ratio of the optically active drug to the optical isomerization inhibitor is 30:1 through 1:200.

**11.** The pharmaceutical composition according to any of claims 2 through 10 wherein the optical isomerization inhibitor is tocopherol.

12. The pharmaceutical composition according to any of claims 2 through 11 which contains polyethylene glycol.

13. A method of inhibiting the optical isomerization of an optically active drug which comprises adding an antioxidant to a pharmaceutical composition.

14. A process for producing a pharmaceutical composition characterized by mixing the optical isomerization inhibitor of claim 1 with an optically active drug.

## INTERNATIONAL SEARCH REPORT

International application No.
PCT/JP94/00261

**A. CLASSIFICATION OF SUBJECT MATTER**

Int. $Cl^5$ A61K47/02, 47/20, 47/22, 47/34, A61K31/55 // C07D487/06, 498/06

According to International Patent Classification (IPC) or to both national classification and IPC

**B. FIELDS SEARCHED**

Minimum documentation searched (classification system followed by classification symbols)

Int. $Cl^5$ A61K47/00-47/34, A61K31/55, C07D487/06, 498/06

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP, A, 57-80328 (Sumitomo Chemical Co., Ltd.), May 19, 1982 (19. 05. 82), & EP, A, 51962 & US, A, 4582845 | 1, 2, 8-10, 12-14 |
| X | JP, A, 1-9285 (Shiseido Co., Ltd.), January 12, 1989 (12. 01. 89), (Family: none) | 1, 2, 8-14 |
| X | JP, A, 56-127309 (Daiichi Seiyaku Co., Ltd.), October 6, 1981 (06. 10. 81), (Family: none) | 1, 2, 8-10, 12-14 |
| X | JP, B1, 36-14048 (N.V. Philips' Gloeilampen-fabrieken), August 22, 1961 (22. 08. 61), (Family: none) | 1, 2, 8-14 |
| X | JP, A, 2-111774 (Fujisawa Pharmaceutical Co., Ltd.), April 24, 1990 (24. 04. 90) & EP, A, 360079 & US, A, 491847 | 1-14 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| April 12, 1994 (12. 04. 94) | May 10, 1994 (10. 05. 94) |
| Name and mailing address of the ISA/ Japanese Patent Office | Authorized officer |
| Facsimile No. | Telephone No. |

Form PCT/ISA/210 (second sheet) (July 1992)